**Europäisches Patentamt**

**European Patent Office**

**Office européen des brevets**

(19)

(11) Veröffentlichungsnummer: **0 021 355**
**A1**

(12) ## EUROPÄISCHE PATENTANMELDUNG

(21) Anmeldenummer: **80103431.5**

(22) Anmeldetag: **19.06.80**

(51) Int. Cl.³: **A 61 B 6/00**

(30) Priorität: **22.06.79 DE 2925274**

(43) Veröffentlichungstag der Anmeldung: **07.01.81**
**Patentblatt 81/1**

(84) Benannte Vertragsstaaten: **BE DE FR GB IT**

(71) Anmelder: **SIEMENS AKTIENGESELLSCHAFT Berlin und München, Postfach 22 02 61, D-8000 München 22 (DE)**

(72) Erfinder: **Wilkens, Achim, Atzelbergerstrasse 14 B, D-8521 Langensendelbach-Bräuningshof (DE)**

(54) **Röntgendiagnostikanlage für angiographische Röntgenaufnahmeserien.**

(57) Die Röntgendiagnostikanlage zur Anfertigung angiographischer Röntgenaufnahmeserien entsprechend dem Kontrastmittelverlauf im Gefäßsystem umfaßt eine Patientenlagerstatt, eine Röntgenröhre mit einer Blendeneinrichtung (7) mit Blendenplatten (8, 9, 10) für die Begrenzung des den Patienten durchstrahlenden Röntgenstrahlenbündels und einen Kassettenwechsler für Röntgenfilmkassetten. Der Kassettenwechsler ist ein Großkassettenwechsler für den gesamten zu erfassenden Bereich. Die Blendeneinrichtung (7) weist eine feste Blende (8) auf, die die maximale Größe des Röntgenstrahlenbündels (6) begrenzt, sowie Mittel (11, 12) zur individuellen, unabhängigen Verstellung von die Abmessung des Röntgenstrahlenbündels (6) in Längsrichtung der Patientenlagerstatt (1) festlegenden Blendenplatten (9, 10).

SIEMENS AKTIENGESELLSCHAFT          Unser Zeichen
Berlin und München                  VPA 79 P 5058  EUR

Röntgendiagnostikanlage für angiographische Röntgenaufnahmeserien

Die Erfindung betrifft eine Röntgendiagnostikanlage zur
Anfertigung angiographischer Röntgenaufnahmeserien entsprechend dem Kontrastmittelverlauf im Gefäßsystem mit
einer Patientenlagerstatt, einer Röntgenröhre mit einer Blendeneinrichtung mit Blendenplatten für die Begrenzung des den Patienten durchstrahlenden Röntgenstrahlenbündels und einem Kassettenwechsler für Röntgenfilmkassetten.

Eine Röntgendiagnostikanlage dieser Art ist z.B. in
der DE-OS 19 30 282 beschrieben. Bei dieser Röntgendiagnostikanlage sind mit dem Kassettenwechsler nur
Röntgenaufnahmen mit einem verhältnismäßig kleinen
Format anzufertigen. Für die Verfolgung des Kontrastmittels, beispielsweise vom Abdomenbereich in die
Beine, ist es daher erforderlich, die Patientenlagerstatt mit dem Patienten in Längsrichtung zu verschieben. Prinzipiell ist es auch möglich, auf die Längs-

Tp 5 Ler / 5.6.1979

verschiebung der Patientenlagerstatt zu verzichten, wenn ein Kassettenwechsler mit Kassetten verwendet wird, dessen Größe den gesamten interessierenden Bereich, beispielsweise von Abdomen bis zu den Füßen, überdeckt. In diesem Fall ist es jedoch wichtig, dafür zu sorgen, daß der Patient nicht unnötiger Strahlung ausgesetzt wird.

Der Erfindung liegt die Aufgabe zugrunde, eine Röntgendiagnostikanlage der eingangs genannten Art zu schaffen, bei der, ausgehend von dem Gedanken der Verwendung von Großkassetten, die den gesamten interessierenden Bereich auf einmal erfassen, der Patient nur bildgebender Strahlung ausgesetzt wird.

Diese Aufgabe ist erfindungsgemäß dadurch gelöst, daß der Kassettenwechsler ein Großkassettenwechsler für Großkassetten für den gesamten zu erfassenden Bereich ist und daß die Blendeneinrichtung eine feste Blende aufweist, die die maximale Größe des Röntgenstrahlenbündels begrenzt, sowie Mittel zur individuellen, unabhängigen Verstellung von die Abmessungen des Röntgenstrahlenbündels in Längsrichtung der Patientenlagerstatt festlegenden Blendenplatten besitzt. Bei der erfindungsgemäßen Röntgendiagnostikanlage ist es möglich, das Röntgenstrahlenbündel in Längsrichtung der Patientenlagerstatt hinsichtlich seiner Einblendung auf das jeweils gewünschte Aufnahmefeld exakt anzupassen und diesem Aufnahmefeld entsprechend auch den Film in einer Großkassette auszuwählen, so daß der Patient nur von bildgebender Strahlung durchdrungen wird. Für die Verfolgung eines Kontrastmittels ist dabei keine Tischlängsverschiebung, sondern lediglich ein Kassettenwechsel erforderlich, wenn in jeder Kassette der Film dem gewünschten Aufnahmefeld entsprechend positioniert und in seiner Größe entsprechend gewählt wird.

Einzelheiten der Erfindung ergeben sich aus den Unteransprüchen.

Die Erfindung ist nachfolgend anhand eines in der
Zeichnung dargestellten Ausführungsbeispiels näher
erläutert. Es zeigen:

Fig. 1 eine Darstellung einer Röntgendiagnostikanlage nach der Erfindung,

Fig. 2 eine beispielsweise Darstellung der Beschickung der Kassetten bei der Röntgendiagnostikanlage gemäß Figur 1, und

Fig. 3 eine für die Erfindung wesentliche Einzelheit der Röntgendiagnostikanlage gemäß
Figur 1.

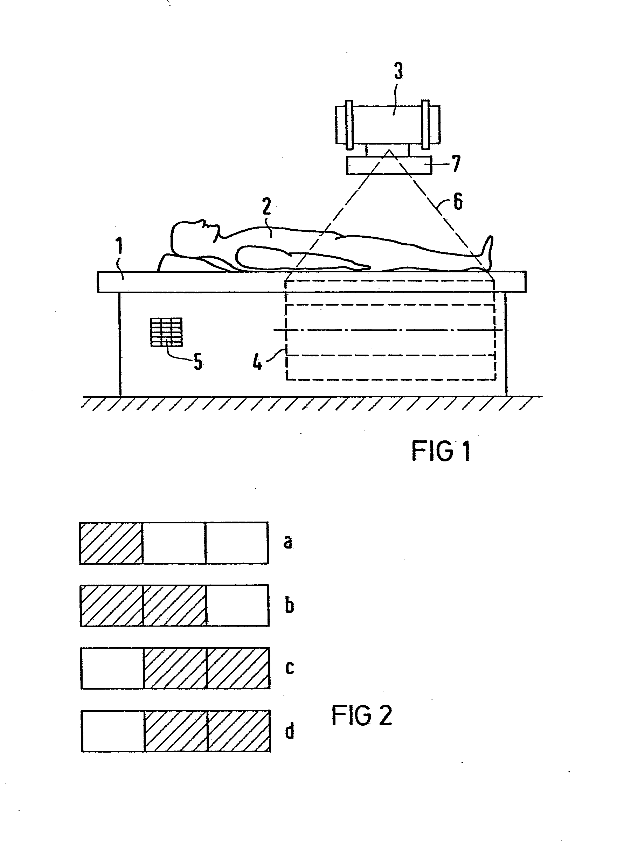

In der Figur 1 ist eine Patientenlagerstatt 1 dargestellt, auf der ein Patient 2 ruht. Angiographische
Aufnahmen vom Patienten 2 werden durch eine Röntgenröhre 3 in Verbindung mit einem Kassettenwechsler 4
in dem Abdomen-Fußbereich angefertigt. Der Kassettenwechsler 4 besteht aus einer Trommel mit sechseckigem
Querschnitt, wobei auf jeder seiner Außenflächen je
eine Großkassette angeordnet werden kann. Maximal
sind also für die Verfolgung des Kontrastmittels im
Patienten 2 sechs Kassetten anwendbar.

Die Figur 2 zeigt beispielsweise die Beschickung von
vier Kassetten im Kassettenwechsler 4 mit Filmen. Jede Kassette ist in ihrer Längsrichtung in drei Be-

0021355

reichen mit je einem Film beschickbar. Bei dem Beispiel a in Figur 2 ist der von links gesehen erste Bereich mit einem Film beschickt, bei dem Beispiel b die ersten beiden Bereiche und bei den Beispielen c und d die letzten beiden Bereiche. Der Figur 2 liegt zugrunde, daß es für dieses Beispiel ausreicht, insgesamt vier Aufnahmen fortschreitend vom Abdomen bis zum Fuß des Patienten 2 anzufertigen. Zwei Außenflächen des Kassettenwechslers 4 werden bei diesem Beispiel also nicht mit Großkassetten bestückt.

Der Aufnahmeablauf erfolgt bei dem Beispiel gemäß Figur 2 so, daß zunächst die den Fällen a bis d der Figur 2 entsprechenden Tasten in einem Tastenfeld 5 gedrückt und damit die Röntgendiagnostikanlage programmiert wird. Anschließend werden vier Großkassetten mit Filmen entsprechend den schraffierten Feldern in den Beispielen a bis d in Figur 2 bestückt und in der Reihenfolge a, b, c, d am Kassettenwechsler 4 befestigt. Nun wird die erste Aufnahme gemäß a gemacht, wobei automatisch das Röntgenstrahlenbündel 6 entsprechend eingeblendet wird. Daraufhin wird der Kassettenwechsler 4 um einen Schritt weiter gedreht und eine Aufnahme gemäß b gemacht, wobei wieder automatisch die Einblendung des Röntgenstrahlenbündels 6 entsprechend verändert wird. Danach erfolgt unter automatischer Veränderung der Einblendung eine Aufnahme gemäß c und schließlich gemäß d. Während des gesamten Aufnahmeablaufes wird die Patientenlagerstatt 1 mit dem Patienten 2 nicht bewegt. Aufgrund der automatischen Veränderung der Einblendung wird immer nur der Bereich des Patienten 2 durchstrahlt, der auch tatsächlich für die Bilderzeugung wichtig ist. Eine unnötige Strahlenbelastung des Patienten erfolgt also nicht.

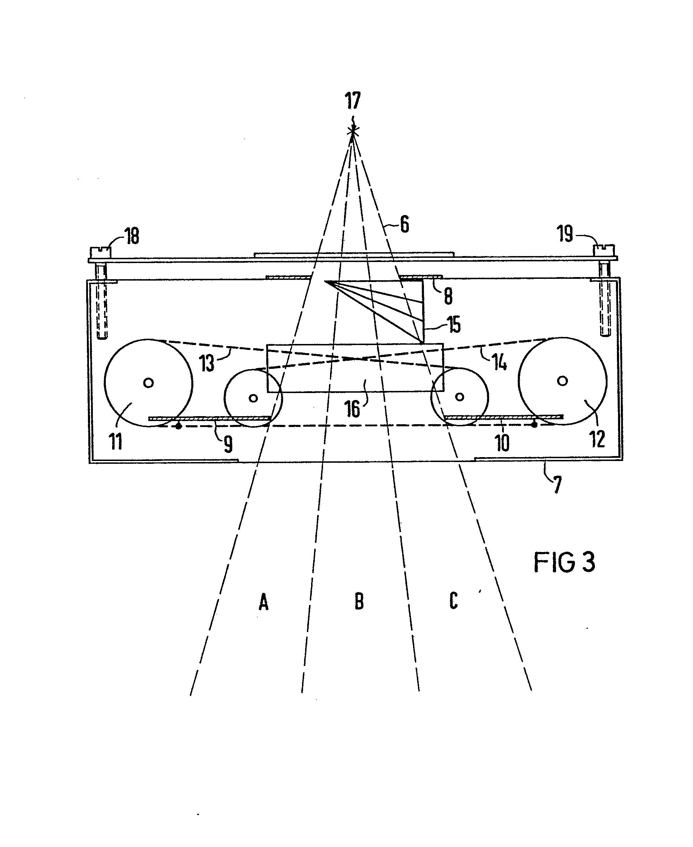

In der Figur 3 ist die Blendeneinrichtung 7 zur Einblendung des Röntgenstrahlenbündels 6 genauer dargestellt. Aus der Figur 3 geht hervor, daß die Blenden-einrichtung 7 eine feste Blende 8 aufweist, die die maximale Größe des Röntgenstrahlenbündels 6 begrenzt. Ferner sind zwei in Längsrichtung der Patientenlagerstatt 1 verstellbare Blendenplatten 9 und 10 vorhanden, die individuell, d.h. unabhängig voneinander, durch je einen Motor 11 bzw. 12 über zugeordnete Seilzüge 13 bzw. 14 verstellbar sind. Die Steuerung der Motoren 11 und 12 erfolgt über die Tasten im Tastenfeld 5, d.h. bei der Wahl der jeweiligen Strahlungsfelder wird das Strahlenbündel 6 automatisch eingeblendet. In der Figur 3 sind die drei Teilfelder A, B, C dargestellt. Für das Beispiel a in Figur 2 wird das Feld A, für das Beispiel b werden die Felder A und B, für das Beispiel d die Felder B und C und für das Beispiel d ebenfalls die Felder B und C eingeblendet.

Zum Ausgleich der abnehmenden Objektdicke beim Übergang von Abdomen auf die Beine des Patienten 2 ist zwischen den Blendenplatten 9 und 10 sowie der festen Blende 6 ein Keilfilterwechsler 15 angeordnet. Durch den Keilfilterwechsler können wahlweise unterschiedliche, keilförmig verlaufende Filter für die Röntgen-strahlung entsprechend der Objektdickenabnahme einge-bracht werden. Eine Berücksichtigung der abnehmenden Objektdicke beim Übergang von Aufnahmen im Abdomenbereich auf Aufnahmen im Beinbereich kann auch dadurch erfolgen, daß eine Einrichtung zur Umschaltung der Röntgenröhrenspannung in Abhängigkeit von den am Tastenfeld 5 gewählten Abmessungen des Röntgenstrahlenbündels vorgesehen wird, wie sie im Prinzip in der DE-OS 19 30 282 beschrieben ist. Der Objektdickenausgleich kann auch gemeinsam durch Keilfilter und entsprechend geringere Röntgenröhrenspannungsabsenkung erfolgen.

Zwischen der festen Blende 8 und den verstellbaren Blendenplatten 9 und 10 ist der Spiegel 16 eines Lichtvisiers angeordnet. Der Spiegel 16 kann strahlendurchlässig sein und das Lichtvisier kann beispielsweise gemäß der US-PS 40 60 733 ausgebildet sein.

Der Abstand der Blendeneinrichtung 7 vom Fokus 17 der Röntgenröhre 3 ist über Schrauben 18, 19 im Röntgenröhrengehäuse einstellbar. Dadurch können verschiedene Film-Fokus-Abstände berücksichtigt werden.

Patentansprüche

1. Röntgendiagnostikanlage zur Anfertigung angiographischer Röntgenaufnahmeserien entsprechend dem Kontrastmittelverlauf im Gefäßsystem mit einer Patientenlagerstatt, einer Röntgenröhre mit einer Blendeneinrichtung mit Blendenplatten für die Begrenzung des den Patienten durchstrahlenden Röntgenstrahlenbündels und einem Kassettenwechsler für Röntgenfilmkassetten,   d a - d u r c h   g e k e n n z e i c h n e t ,   daß der Kassettenwechsler (4) ein Großkassettenwechsler für den gesamten zu erfassenden Bereich ist und daß die Blendeneinrichtung (7) eine feste Blende (8) aufweist, die die maximale Größe des Röntgenstrahlenbündels (6) begrenzt, sowie Mittel (11, 12) zur individuellen, unabhängigen Verstellung von die Abmessung des Röntgenstrahlenbündels (6) in Längsrichtung der Patientenlagerstatt festlegenden Blendenplatten (9, 10) besitzt.

2. Röntgendiagnostikanlage nach Anspruch 1,   d a - d u r c h   g e k e n n z e i c h n e t ,   daß in der Blendeneinrichtung (7) fokusnah ein Keilfilterwechsler (15) zum Objektdickenausgleich angeordnet ist.

3. Röntgendiagnostikanlage nach Anspruch 1 oder 2,   d a d u r c h   g e k e n n z e i c h n e t ,   daß eine Einrichtung zur Umschaltung der Röntgenröhrenspannung in Abhängigkeit von den gewählten Abmessungen des Röntgenstrahlenbündels (6) vorhanden ist.

4. Röntgendiagnostikanlage nach einem der Ansprüche 1 bis 3,   d a d u r c h   g e k e n n z e i c h - n e t ,   daß zwischen der festen Blende (8) und den verstellbaren Blendenplatten (9, 10) der Spiegel (16) eines Lichtvisiers angeordnet ist.

5. Röntgendiagnostikanlage nach einem der Ansprüche 1 bis 4, dadurch gekennzeichnet, daß Mittel (18, 19) zur Einstellung des Abstandes der Blendeneinrichtung (7) vom Fokus (13) vorhanden sind.

**FIG 1**

**FIG 2**

FIG 3

**Europäisches Patentamt**

**EUROPÄISCHER RECHERCHENBERICHT**

0021355

Nummer der Anmeldung

EP 80103431.5

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - B2 - 1 911 186 (GÄRTNER) <br> + Gesamt + <br> -- | 1 |
| | US - A - 4 097 748 (MONVOISIN) <br> + Gesamt + <br> -- | 1 |
| | DE - A1 - 2 409 154 (PHILIPS) <br> + Gesamt + <br> -- | 1,3 |
| | DE - B2 - 2 200 816 (SIEMENS) <br> + Gesamt + <br> -- | 4 |
| | US - A - 3 920 997 (MUNCH) <br> + Gesamt + <br> -- | 1 |
| D | DE - A - 1 930 282 (SIEMENS) <br> + Gesamt + <br> -- | 1,3 |
| D | US - A - 4 060 733 (FRANKE) <br> + Gesamt + <br> ---- | 4 |

**KLASSIFIKATION DER ANMELDUNG (Int.Cl. 3)**

A 61 B 6/00

**RECHERCHIERTE SACHGEBIETE (Int. Cl. 3)**

A 61 B   6/00
G 01 N 23/00
G 03 B 41/00
H 05 G   1/00

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie,   übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| WIEN | 15-09-1980 | SCHMIDT |

EPA form 1503.1   06.78